Europäisches Patentamt
European Patent Office
Office européen des brevets

(19)

(11) Veröffentlichungsnummer: **0 356 418 A2**

(12) **EUROPÄISCHE PATENTANMELDUNG**


(21) Anmeldenummer: **89890205.1**

(22) Anmeldetag: **31.07.89**

(51) Int. Cl.[5]: **C 12 Q 1/26**
C 12 Q 1/54, C 12 M 1/40, G 01 N 21/64

(30) Priorität: **24.08.88 AT 2093/88**

(43) Veröffentlichungstag der Anmeldung: **28.02.90 Patentblatt 90/09**

(84) Benannte Vertragsstaaten: **DE FR GB**



(71) Anmelder: **AVL AG**
**P.O. Box 10 Grabenstrasse 11**
**CH-8201 Schaffhausen (CH)**

(72) Erfinder: **Wolfbeis, Otto S., Dr.**
**Im Hoffeld 32**
**A-8046 Graz (AT)**

**Trettnak, Wolfgang, Mag.rer.nat.**
**Neudorf 21**
**A-8562 Mooskirchen (AT)**

(74) Vertreter: **Krause, Walter, Dr. Dipl.-Ing. et al**
**Postfach 200 Singerstrasse 8**
**A-1014 Wien (AT)**


(54) **Verfahren zur Bestimmung der Konzentration eines Enzymsubstrates und Sensor zur Durchführung des Verfahrens.**


(57) Für ein Verfahren zur Bestimmung der Konzentration eines Enzymsubstrates - insbesondere von Glucose - in einer Probe, wobei die Probe mit einem entsprechenden Enzym in Kontakt gebracht und dessen Fluoreszenzspektrum nach Fluoreszenzanregung gemessen wird und eine Änderung des Fluoreszenzspektrums als Maß für die Änderung der Konzentration des Enzymsubstrates herangezogen wird, wird vorgeschlagen, daß zur kontinuierlichen, reversiblen Bestimmung der Konzentration des Enzymsubstrates ein Enzym aus der Gruppe der Oxydasen und Oxygenasen mit einem daran gebundenen Flavincoenzym (FMN, FAD) verwendet wird, welches durch das Enzymsubstrat in die reduzierte Form und durch in der Probe gelösten molekularen Sauerstoff in die oxidierte Form gebracht wird, wobei die durch die Reduktion des Flavincoenzyms auftretende Änderung des Fluoreszenzspektrums gemessen wird.


Fig. 2

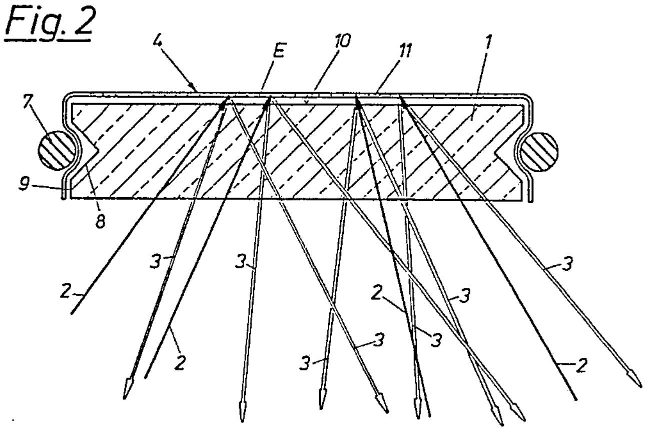


EP 0 356 418 A2

**Beschreibung**

## Verfahren zur Bestimmung der Konzentration eines Enzymsubstrates und Sensor zur Durchführung des Verfahrens

Die Erfindung betrifft ein Verfahren zur Bestimmung der Konzentration eines Enzymsubstrates in einer Probe, wobei die Probe mit einem entsprechenden Enzym in Kontakt gebracht und dessen Fluoreszenzspektrum nach Fluoreszenzanregung gemessen wird und eine Änderung des Fluoreszenzspektrums als Maß für die Änderung der Konzentration des Enzymsubstrates herangezogen wird, sowie einen optischen Sensor zur Durchführung des Verfahrens.

Die Bestimmung von Enzymsubstraten, beispielsweise von Glucose spielt eine bedeutende Rolle in der klinischen Diagnose, da sie umfassende Aussagen über das Vorliegen von Stoffwechselstörungen (z.B. bei Diabetes mellitus) erlaubt. Daneben wird Glucose auch häufig im Rahmen der Qualitätskontrollen der Nahrungsmittelindustrie sowie in der Biotechnologie bestimmt, da die gängigen Bioreaktoren eine kontinuierliche Versorgung mit Nährstoffen, speziell mit Glucose, erfordern. Glucose und andere Sensoren für Enzymsubstrate spielen dabei eine besondere Rolle, weil eine kontinuierliche und reversible Erfassung der Enzymsubstratkonzentrationen erwünscht ist. Ein verläßlicher Glucosesensor ist beispielsweise zur Zeit noch die limitierende Größe bei der Entwicklung eines künstlichen Pankreas.

Eine große Vielfalt von Bestimmungsmethoden in diesem Zusammenhang ist bereits bekannt geworden. Man unterscheidet dabei kontinuierliche und diskontinuierliche Verfahren. Die diskontinuierlichen Verfahren haben den Nachteil, daß sie unter Zerstörung der Probe durchgeführt werden müssen, und daß sie experimentelle Bedingungen erfordern, welche beispielsweise für in-vivo-Applikationen ungeeignet sind (Zugabe agressiver Reagenzien, Arbeiten in Gegenwart starker Säuren oder Laugen). Ein weiterer Nachteil der diskontinuierlichen Methode besteht darin, daß zur Erlangung einer kontinuierlichen Überwachung eine ständige Probennahme in entsprechend kurzen Zeitabständen erforderlich ist. Aus diesem Grund hat man sich in letzter Zeit bevorzugt mit der Entwicklung kontinuierlicher Sensoren für Glucose beschäftigt. Man unterscheidet dabei zwei große Gruppen, nämlich die elektrochemischen und die optischen Biosensoren für Glucose.

Ein Überblick über den Stand der Technik findet sich in "Biosensors: Fundamental and Applications", Oxford University Press, Oxford (1987) von H. P. F. Turner, I. Karube und G. Wilson. Daraus ist ersichtlich, daß die elektrochemischen Sensoren für Glucose (und andere klinisch relevante Enzymsubstrate) einen hohen Stand der Kunst erreicht haben. Allerdings sind elektrochemische Biosensoren mit verschiedenen Nachteilen behaftet. Eines der größten Probleme bei elektrochemischen Sensoren besteht darin, daß die notwendige Elektrolytbrücke sehr anfällig ist, und Anlaß zu kontinuierlichen Drifts gibt. Weiters können elektrochemische Sensoren nur über kurze Distanzen betrieben werden, weil sie eine elektrische Verbindung zum Probenraum herstellen, was besonders bei in-vivo-Studien auch ein gewisses Risiko darstellt. Im Falle der Bioreaktoren ist es nachteilig, daß sich elektrochemische Sensoren nur sehr schlecht sterilisieren lassen.

Man hat in letzter Zeit nach Alternativen zu elektrochemischen Sensoren gesucht und sie in den optischen, speziell in den faseroptischen, Sensoren gefunden. Optische Sensoren benötigen im Gegensatz zu elektrochemischen Sensoren keine Referenzelektrode, erlauben den Transport von Licht über große Distanzen und bilden keine elektrische Verbindung zum Probenobjekt, sodaß sie auch nicht durch statische Potentiale oder Körperpotentiale beeinflußt werden. Bei den bekannt gewordenen optischen Biosensoren für Glucose und ähnliche enzymatische abbaubare Substrate kann man zwei Arten unterscheiden:

Die erste besteht darin, daß man eine enzymatische Reaktion ablaufen läßt, während der eine Spezies gebildet wird, welche mit einem Transducerelement detektiert werden kann. Beispielsweise verläuft die Oxidation von Glucose, durch das Enzym Glucoseoxydase (GOD), nach folgender chemischer Reaktion:

$$\text{Glucose} + O_2 \xrightarrow{GOD} \text{Gluconsäure} + H_2O_2 + H^+$$

Man hat somit die Möglichkeit, durch Messung des Verbrauches von Sauerstoff oder der Bildung von $H_2O_2$ oder des Abfalles im pH-Wert auf die vorhandene Glucosekonzentration zurückzuschließen, da alle diese Parameter sich parallel mit der Veränderung der Glucosekonzentration ändern. Eine optische Anordnung zur Messung von Glucose, welche auf der Bestimmung des verbrauchten Sauerstoffes beruht, wird in Adv. Exp. Med. Biol. 75, 65 (1976) bzw. in der DE-PS 2.948.904 beschrieben. In Analytical Biochem. 138, 430 (1984) ist ein Glucosesensor geoffenbart, welcher auf der Messung der gebildeten Säure ($H^+$) durch die Verfolgung der Farbänderungen eines zugesetzten pH-Indikators beruht. Ein kontinuierlicher Sensor für Glucose, der auf Messung des gebildeten $H_2O_2$ beruht, ist bisher nicht bekannt geworden.

Ein anderes Prinzip zur Glucosekonzentrationsbestimmung wird in einem Affinitätssensor der US-PS 4,344,438 beschrieben. Es handelt sich dabei um einen optischen Sensor für alle Arten von Plasmabestandteilen, welche in der Lage sind, eine kompetitive Bindung an einer Rezeptorseite in einer Meßkammer am Ende

eines Lichtleiters einzugehen. Der kompetitive Ligand ist fluoreszenzmarkiert und wird je nach der Menge an Liganden in diese Kammer verdrängt, worauf das absorbierte oder emittierte Licht vom Lichtleiter gesehen wird. Ein typisches Beispiel besteht darin, daß man Concanavalin A auf Sepharose immobilisiert und Fluorescein-markiertes Dextran an das Concanavalin binden läßt. Glucose ist in der Lage, das markierte Dextran zu verdrängen, worauf das fluoreszenzmarkierte Dextran vom Lichtleiter gesehen wird und als Ergebnis ein Anstieg in der Fluoreszenzintensität beobachtet wird.

Ein Verfahren der eingangs genannten Art wird in Anal. Chem. 60, 1080 (1988) von Wangsa und Arnold beschrieben, und zwar speziell für die Bestimmung von Lactat. Lactat wird dabei durch das Enzym Lactatdehydrogenase in Pyruvat überführt, gleichzeitig wird das nicht gebundene $NAD^+$ des Enzymes in das stark fluoreszierende NADH überführt. Durch Messung der Zunahme der Fluoreszenz des NADH wird auf die vorhandene Konzentration an Lactat geschlossen. Dieser Sensor ist allerdings mit verschiedenen Nachteilen behaftet. Als größter Nachteil kann gelten, daß er nicht reversibel ist, da die Reaktion zu Ende kommt, wenn alles $NAD^+$ verbraucht ist. Aus diesem Grund muß der Reaktion ständig frisches $NAD^+$ zugegeben werden. Dies ist ein genereller Nachteil aller Biosensoren, welche auf der Messung der intrinsischen Fluoreszenz des NADH beruhen. Somit handelt es sich nicht um einen Sensor im engeren Sinn, da er die Bedingung einer vollkommenen Reversibilität nicht erfüllt.

Als weiterer Nachteil ist die zur Fluoreszenz-Anregung von NADH erforderliche UV-Wellenlänge von 350 nm anzusehen, da sowohl Glasfasern als auch Plastikfasern dieses Licht nicht oder nur sehr schlecht leiten und zudem die benötigten Lichtquellen relativ teuer sind.

Alle genannten Verfahren bzw. Sensoren haben den Nachteil, daß sie entweder irreversibel arbeiten oder einen relativ komplexen Sensoraufbau benötigen, im wesentlichen eine enzymatisch aktive Schicht und dazu ein Transducerelement wie z.B. eine Sauerstoff oder pH-Optode aufweisen oder, wie im Falle des Glucosesensors auf Basis der kompetitiven Bindung, eine kleine aber relativ komplizierte Meßkammer mit mehreren Arten von Immobilisierungsschritten benötigen.

Aufgabe der Erfindung ist es, ein einfaches Verfahren zur Bestimmung von Konzentrationen eines Enzymsubstrates in einer Probe sowie einen optischen Sensor zur Durchführung dieses Verfahrens vorzuschlagen, wobei insbesondere ein reversibler Sensor zur kontinuierlichen Glucosebestimmung erhalten werden soll.

Diese Aufgabe wird erfindungsgemäß dadurch gelöst, daß zur kontinuierlichen, reversiblen Bestimmung der Konzentration des Enzymsubstrates ein Enzym aus der Gruppe der Oxydasen und Oxygenasen mit einem daran gebundenen Flavincoenzym (FMN,FAD) verwendet wird, welches durch das Enzymsubstrat in die reduzierte Form und durch in der Probe gelösten molekularen Sauerstoff in die oxidierte Form gebracht wird, wobei die durch die Reduktion des Flavincoenzyms auftretende Änderung der intrinsischen Fluoreszenz des Flavincoenzyms gemessen wird.

Das erfindungsgemäße Verfahren beruht auf der überraschenden Beobachtung, daß sich die Eigenfluoreszenz von Enzymen, welche als Cofaktor ein Coenzym aus der Gruppe der Flavine besitzen, während der enzymatischen Aktivität in charakteristischer Weise ändert und zwar entgegen herrschenden Lehrmeinungen, wonach GOD nicht fluoreszieren bzw. die Fluoreszenz des FAD im Enzym durch das Protein gelöscht werden soll. Es kann beim erfindungsgemäßen Verfahren zu einem Abfall oder zu einem Anstieg in der Fluoreszenzintensität kommen. Diese Fluoresenzänderung ist in Gegenwart von Sauerstoff vollkommen reversibel und kann somit als direkte optische Information für einen optischen Biosensor verwendet werden. Die Verwendung eines Transducerelementes wie z.B. eines Sauerstoff- oder pH-Sensors ist somit nicht mehr erforderlich. Weiters liegen die Anregungswellenlängen von Flavoproteinen in einem Spektralbereich von typischerweise 400 bis 500 nm und sind somit für die Verwendung gängiger Glas- bzw. Plastiklichtleiter geeignet. Zusätzlich ist durch die langwellige Emission bei über 500 nm eine große Stokes'sche Verschiebung gegeben, sodaß auch die optische Separation von Anregungs- und Fluoreszenzstrahlung einfach ist.

Es ist bekannt, daß bei Flavoenzymen die Redoxreaktion an einem Flavincoenzym abläuft. Als Flavincoenzyme bezeichnet man solche Coenzyme, welche ein Flavinmononukleotid (FMN) oder ein Flavinadenindinukleotid (FAD) besitzen. Bei der enzymatischen Oxidation geht der Cofaktor gleichzeitig in eine reduzierte Form über, welche dann durch Sauerstoff (als zweites Substrat) unmittelbar wieder in die oxidierte Form überführt wird. Der Übergang von oxidierter Form zu reduzierter Form ist mit einer Änderung der Fluoreszenzeigenschaften verbunden, welche als analytische Information dient.

In einer Ausgestaltung der Erfindung ist vorgesehen,daß zur Bestimmung der Glucosekonzentration eine Glucoseoxydase mit einem Flavinadenindinukleotid (FAD) verwendet wird. Die folgenden beiden Gleichungen geben das zweistufige Reaktionsschema wieder:

$$\text{Glucose} + \text{GOD-FAD} \rightarrow \text{Gluconolacton} + \text{GOD-FADH}_2$$

$$\text{GOD-FADH}_2 + O_2 \rightarrow \text{GOD-FAD} + H_2O_2$$

Es ist erfindungsgemäß auch möglich, die Alkohol- bzw. Glutaminsäurekonzentration mit Hilfe des Enzyms Alkoholoxydase bzw. Glutaminsäureoxydase mit einem Flavincoenzym zu bestimmen.

Das erfindungsgemäße Verfahren ist auf alle enzymatischen Reaktionen anwendbar, bei denen FMN oder FAD als Coenzym auftreten und Sauerstoff in der Probe vorhanden ist. FMN und FAD sind vorzugweise in Enzymen aus der Gruppe der Oxydasen und Oxygenasen vorhanden. Typische Beispiele solcher Enzyme (mit Enzyme Classification) sind:

Glucoseoxydase (EC 1.1.3.4), Galactoseoxydase (EC 1.1.3.9), Lactatoxydase (EC 1.1.3.2), Lactatmonooxygenase (EC 1.13.12.4), Xanthinoxydase (EC 1.2.3.2), Aspartatoxydase (EC 1.4.3.1), L-Aminosäureoxydase (EC

1.4.3.2), Bilirubinoxydase (EC 1.3.3.5), Cholesterinoxydase (EC 1.1.3.6), Monoaminoxydase (EC 1.4.3.4), Thiaminoxydase (EC 1.4.3.6), Uratoxydase (EC 1.7.3.3), Sulfitoxydase (EC 1.8.3.1), Cytochromoxydase (EC 1.9.3.1), Lipoxygenase (EC 1.13.1.13), Alkoholoxydase (EC 1.1.3.13) und verschiedene andere Enzyme dieser Art.

Trägt man die Fluoreszenzintensität in Abhängigkeit von der Konzentration des Enzymsubstrates auf (siehe auch Fig. 5), so stellt man einen Bereich fest, in welchem geringe Konzentrationszuwächse einen großen Anstieg der Fluoreszenzintensität verursachen. Der Sensor ist somit in diesem Bereich sehr sensibel. Erfindungsgemäß kann hier vorgesehen sein, daß der Probe vor deren Messung molekularer Sauerstoff in bekannter Konzentration zugeführt wird, sodaß jener Bereich der Ansprechfunktion, in welchem geringe Konzentrationsänderungen des Enzymsubstrates zu großen Änderungen der Fluoreszenzin tensität führen, in einen vorgebbaren Konzentrationsbereich verschoben wird. Angewandt kann dieses Verfahren beispielsweise in Bioreaktoren werden, wo in bestimmten Konzentrationsbereichen genaue Messungen notwendig sind.

Eine weitere Möglichkeit zur Steuerung des Empfindlichkeitsbereiches des Verfahrens besteht darin, vor das Sensormaterial eine sogenannte Diffusionsschwelle einzubauen. Sie bewirkt, daß im Inneren des Sensors eine viel kleinere Konzentration an Substrat herrscht als außen. Eine Verbreiterung des für die Messung günstigen Bereiches der Ansprechkurve ist dadurch möglich.

Ein optischer Sensor zur Bestimmung der Konzentration eines Enzymsubstrates mit einem für das Anregungs- und Fluoreszenzlicht durchlässigen Trägerelement, auf welches probenseitig eine Sensorschicht immobilisiert ist, wird erfindungsgemäß dadurch gekennzeichnet, daß die Sensorschicht ein Enzym aus der Gruppe der Oxydasen und Oxygenasen mit einem daran gebundenen Flavincoenzym aufweist.

Der optische Sensor ist in verschiedenen Ausführungsformen denkbar. So kann erfindungsgemäß das Enzym in einem Enzymgel oder einer Enzymlösung vorliegen, welche(s) durch eine Membran mit einem Ausschlußmolekulargewicht kleiner 30.000 am Trägerelement, beispielsweise einen, planaren Träger, mechanisch immobilisiert ist. Die mechanische Immobilisation kann z.B. dadurch erfolgen, daß das Enzym mit Hilfe einer Cellulosemembran mit einem Ausschlußmolekulargewicht von unter 30.000 zurückgehalten wird, da die meisten Enzyme Molekulargewichte über 60.000 besitzen.

In einer Weiterbildung der Erfindung ist vorgesehen, daß das Enzym in einer Sensorschicht aus Hydrogel oder Polyacrylamid vorliegt. Dabei wird das Enzym durch Einbetten in ein Gel physikalisch immobilisiert.

Weiters ist vorgesehen, daß das Enzym durch chemische Immobilisation direkt an die Oberfläche des Trägerelementes gebunden ist. Die chemische Immobilisation kann entweder auf elektrostatische Weise, z. B. durch Bindung an eine Ionentauschermembran oder durch kovalente Bindung an ein Material wie z. B. quervernetztes Serumalbumin oder an eine Membran wie z. B. Cellulose oder Polyamid erfolgen.

Das FMN- oder FAD-haltige Enzym wird somit zuerst auf ein festes Trägerelement aufgebracht bzw. an seiner Oberfläche mechanisch, physikalisch oder chemisch immobilisiert. Die Enzymschicht ist der Probe zugewandt und die Anregung bzw. die Abnahme der Fluoreszenzstrahlung erfolgt von der Rückseite dieses festen und optisch transparenten Trägerelementes. Um Interferenzen durch die Eigenfluoreszenz des Probenmediums zu verhindern, kann vorgesehen werden, daß die enzymhaltige Sensorschicht an der Oberfläche mit einem optisch nicht transparenten Medium abgedeckt ist, welches aber für das zu bestimmende Substrat durchlässig ist. Solche Materialien sind z. B. mit Aktivkohle dunkelgefärbte Hydrogele. Bei verschieden flüchtigen Enzymsubstraten wie z. B. Alkohol ist auch eine Abdeckung mit einer hydrophoben Schicht wie z. B. schwarzem Teflon oder Silicon möglich.

Schließlich ist erfindungsgemäß vorgesehen, daß das Enzym direkt am Ende eines Lichtleiters immobilisiert ist.

Im folgenden wird die Erfindung anhand von Zeichnungen näher erläutert. Es zeigen:

Fig. 1 und 2 einen optischen Sensor nach der Erfindung in Schnittdarstellung,
Fig. 3a bis c einen am Ende eines Lichtleiters angeordneten Sensor,
Fig. 4 eine Meßvorrichtung mit einem Sensor nach der Erfindung, und
Fig. 5 bis 7 Diagramme von Meßergebnissen.

Die Fig. 1 und 2 zeigen einen optischen Sensor in zwei typischen Anordnungen. Das Trägerlement, beispielsweise ein Plexi glasplättchen, ist jeweils mit 1 bezeichnet, die Anregungs-bzw. Fluoreszenzstrahlung mit 2 bzw. 3.

Bei der in Fig. 1 dargestellten Ausführungsvariante befindet sich die Sensorschicht 4 mit dem Enzym E in einer probenseitigen Ausnehmung 5 des Trägerelementes 1 und liegt als Enzymlösung oder Enzymgel vor, welches durch eine für das Enzym undurchlässige Membran 6 mechanisch immobilisiert wird. Die Membran 6 wird durch einen O-Ring 7 in einer Nut 8 an der Seitenfläche 9 des Trägerelementes 1 festgehalten.

Der optische Sensor nach Fig. 2 weist an der der Probe zugewandten Seite 10 eine die Sensorschicht 4 bildende Enzymmembran 11 auf, welche in der in Fig. 1 beschriebenen Art befestigt ist. Das Enzym E ist auf bzw. in der Membran 11 immobilisiert.

Die Fig. 3a bis c zeigen typische Anordnungsmöglichkeiten, bei welchen das Ende 12 eines Lichtleiters als Trägerelement 1 verwendet wird. Der Kern des Lichtleiters ist mit 13 und dessen Mantel mit 14 bezeichnet. In Fig. 3a befindet sich das Enzym auf einer Enzymmembran 11, welche mit einem O-Ring 7 am Ende 12 des Lichtleiters befestigt ist. In Fig. 3b wird eine Enzymlösung oder ein Enzymgel mit einer Dialysemembran 6 und einem O-Ring 7 in einer Ausnehmung 5 am Lichtleiterende festgehalten. Schließlich wird das Enzym E in Fig. 3c direkt am distalen Ende 15 des Lichtleiters immobilisiert.

In einer anderen denkbaren Ausführung kann das analytische Signal dadurch gewonnen werden, daß das

Enzym nicht am distalen Ende des Lichtleiters immobilisiert wird, sondern direkt auf dem Kern 13 des Lichtleiters, nachdem man die Schutzschicht und den Mantel 14 im Endbereich des Lichtleiters entfernt hat. Man mißt in diesem Fall mit Hilfe der sogenannten evaneszierenden Welle, d. h. des elektrischen Feldvektors, welcher bei der Totalreflexion an der Grenzfläche in die optisch dünnere Phase evanesziert. Der elektrische Feldvektor kann dort innerhalb der Eindringtiefe die Fluoreszenz des Enzyms anregen. Die Fluoreszenzstrahlung koppelt in den optischen Wellenleiter ein und kann an dessen anderem Ende detektiert werden.

Die in Fig. 4 dargestellte Meßvorrichtung mit einem Sensor nach Fig. 3 an einem Probenort P, besteht aus einer Lichtquelle 16, welche in der Lage ist, Licht zwischen 350 und 500 nm zu liefern, einem optischen Filter bzw. Monochromator 17, welcher das Anregungslicht auf einen schmalen Bandbereich einengt, einem Lichtleiterarm 18, welcher das Anregungslicht zum Sensor am Ende 12 des zweiarmigen Lichtleiters führt, und einem zweiten Lichtleiterarm 19, welcher das vom Enzym emittierte Fluoreszenzlicht über einen weiteren Monochromator 20 einem Photodetektor 21 zuführt, einer Verstärkereinrichtung 22 und einem Anzeigeelement 23, welches noch mit einer Auswerteeinrichtung gekoppelt sein kann.

Als Lichtquelle kommen in Frage: Halogenlampen, Xenonlampen (gepulst oder im Dauerbetrieb), lichtemittierende Dioden, Laser und Metalldampflampen. Als optische Filter sind geeignet: Interferenzfilter, Kantenfilter, Prismen oder Gittermonochromatoren. Als Lichtleiter werden vorzugsweise solche verwendet, welche aus Plastik oder Glas sind, da sie nur ein geringes Bruchrisiko aufweisen, was bei invasiven Methoden von großem Vorteil ist. Es können sowohl faseroptische Bündel als auch Einzelfasern verwendet werden. Entsprechende Anordnungen zur Trennung von eingestrahltem Licht und Fluoreszenzlicht sind seit längerem bekannt.

Die vom Sensor emittierte Strahlung besteht aus der eigentlichen Fluoreszenzstrahlung und einem Streulichtanteil, welcher durch elastische oder unelastische Streuung der Anregungsstrahlung in der Sensorzone gebildet wird. Zur Abtrennung des Streulichtes, welches ohne eigentliche analytische Information ist und höchstens als Referenzsignal verwendet wird, ist es vorgesehen, dieses mit Hilfe geeigneter Filter abzutrennen. Solche Filter sind z. B. Monochromatoren, dichroitische Filter oder Steilkantenfilter. Als Lichtdetektoren kommen Photodioden, Phototransistoren oder Photomultiplier in Frage.

Fig. 5 zeigt die Fluoreszenzintensität I in Abhängigkeit von der Glucosekonzentration in mM, gemessen mit einem Sensor nach Fig. 1. Die Messungen erfolgten bei pH = 7 und 22,5° C im Durch fluß. Die Abhängigkeit von der Sauerstoffkonzentration wird durch die beiden mit a und b bezeichneten Kurven gezeigt, wobei die Probenlösuung bei a luftgesättigt und b gesättigt mit einer $O_2/N_2$-Mischung mit 7,88 % $O_2$ ist.

Fig. 6 zeigt die typische Ansprechkurve eines Glucosesensors mit Glucoseoxydase in Lösung (Anordnung nach Fig. 1) auf verschiedenen Glucosekonzentrationen im Durchfluß. Die Messungen erfolgten bei pH = 7, einer Temperatur von 22,5° C und Luftsättigung.

Fig. 7 zeigt die Abhängigkeit des Peak-Integrals PI eines Glucosesensors von der Glucosekonzentration in mM, wobei die Probenlösungen jeweils 65 s durch die Durchflußzelle gepumpt wurden. Als Meßanordnung wurde eine Anordnung nach Fig. 1 gewählt. Die Messung erfolgte bei pH = 7, 22,5° C und Luftsättigung.

Zusammenfassend kann somit gesagt werden, daß der erfindungsgemäße optische Biosensor sich in folgenden Punkten von bisherigen optischen Biosensoren unterscheidet:

1) Er erlaubt eine direkte Bestimmung der Meßgröße, da keine zusätzlichen Indikatoren in die Sensorschicht eingebracht werden müssen und somit kein Transducerelement notwendig ist.

2) Im Gegensatz zu vielen bekannten optischen Biosensoren ist nur ein einziger Reaktionsraum vorhanden, sodaß es nicht zu einem Massentransport von Indikatorraum zu Reaktionsraum kommen muß.

3) Es wird keine Spezies gemessen, die durch chemisch Reaktion erst entsteht oder verbraucht wird, sondern es wird das Enzym selbst vermessen.

4) Der Sensor spricht im Gegensatz zu den auf NADH basierenden Sensoren in vollkommen reversibler Weise an und ist wegen der langwelligen Anregung und Emission mit in der Biomedizin geforderten Plastiklichtleitern kompatibel.

**Patentansprüche**

1. Verfahren zur Bestimmung der Konzentration eines Enzymsubstrates in einer Probe, wobei die Probe mit einem entsprechenden Enzym in Kontakt gebracht und dessen Fluoreszenzspektrum nach Fluoreszenzanregung gemessen wird und eine Änderung des Fluoreszenzspektrums als Maß für die Änderung der Konzentration des Enzymsubstrates herangezogen wird, **dadurch gekennzeichnet**, daß zur kontinuierlichen, reversiblen Bestimmung der Konzentration des Enzymsubstrates ein Enzym aus der Gruppe der Oxydasen und Oxygenasen mit einem daran gebundenen Flavincoenzym (FMN,FAD) verwendet wird, welches durch das Enzymsubstrat in die reduzierte Form und durch in der Probe gelösten molekularen Sauerstoff in die oxidierte Form gebracht wird, wobei die durch die Reduktion des Flavincoenzyms auftretende Änderung der intrinsischen Fluoreszenz des Flavincoenzyms gemessen wird.

2. Verfahren nach Anspruch 1, **dadurch gekennzeichnet**, daß der Probe vor deren Messung molekularer Sauerstoff in bekannter Konzentration zugeführt wird, sodaß jener Bereich der Ansprechfunktion, in welchem geringe Konzentrationsänderungen des Enzymsubstrates zu großen Änderungen

der Fluoreszenzintensität führen, in einen vorgebbaren Konzentrationsbereich verschoben wird.

3. Verfahren nach Anspruch 1 oder 2, **dadurch gekennzeichnet**, daß zur Bestimmung der Glucosekonzentration eine Glucoseoxydase mit einem Flavinadenindinukleotid (FAD) verwendet wird.

4. Verfahren nach Anspruch 1 oder 2, **dadurch gekennzeichnet**, daß zur Bestimmung der Alkoholkonzentration eine Alkoholoxydase mit einem Flavinadenindinuleotid (FAD) als Coenzym verwendet wird.

5. Verfahren nach Anspruch 1 oder 2, **dadurch gekennzeichnet**, daß zur Bestimmung der Glutaminsäurekonzentration eine Glutaminsäureoxydase mit einem Flavincoenzym verwendet wird.

6. Optischer Sensor zur Bestimmung der Konzentration eines Enzymsubstrates mit einem für das Anregungs- und Fluoreszenzlicht durchlässigen Trägerelement, auf welches probenseitig eine Sensorschicht immobilisiert ist, **dadurch gekennzeichnet**, daß die Sensorschicht (4) ein Enzym (E) aus der Gruppe der Oxydasen und Oxygenasen mit einem daran gebundenen Flavincoenzym aufweist.

7. Optischer Sensor nach Anspruch 6, **dadurch gekennzeichnet**, daß das Enzym (E) in einem Enzymgel oder einer Enzymlösung vorliegt, welche(s) durch eine Membran (6) mit einem Ausschlußmolekulargewicht kleiner 30.000 am Trägerelement (1) mechanisch immobilisiert ist.

8. Optischer Sensor nach Anspruch 6, **dadurch gekennzeichnet**, daß das Enzym (E) in einer Sensorschicht (4) aus Hydrogel oder Polyacrylamid vorliegt.

9. Optischer Sensor nach Anspruch 6, **dadurch gekennzeichnet**, daß das Enzym (E) durch chemische Immobilisation direkt an die Oberfläche (15) des Trägerelementes (1) gebunden ist.

10. Optischer Sensor nach einem der Ansprüche 6 bis 9, **dadurch gekennzeichnet**, daß das Enzym (E) direkt am Ende (12) eines Lichtleiters immobilisiert ist.

Fig. 1

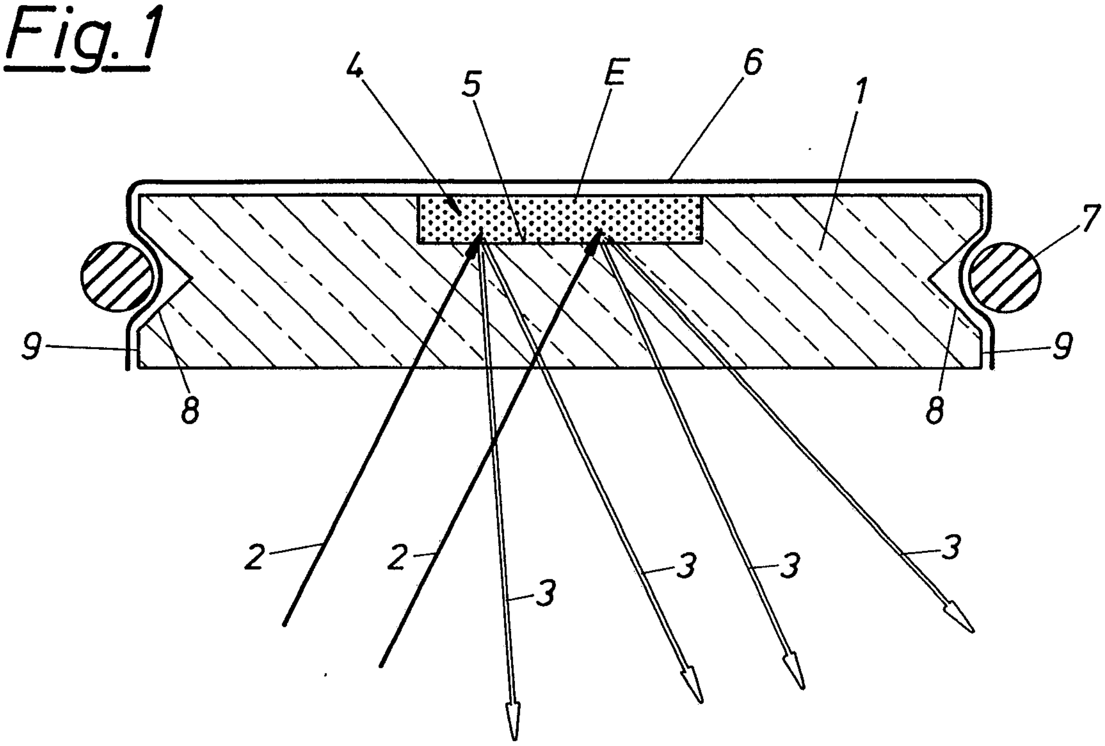


Fig. 2

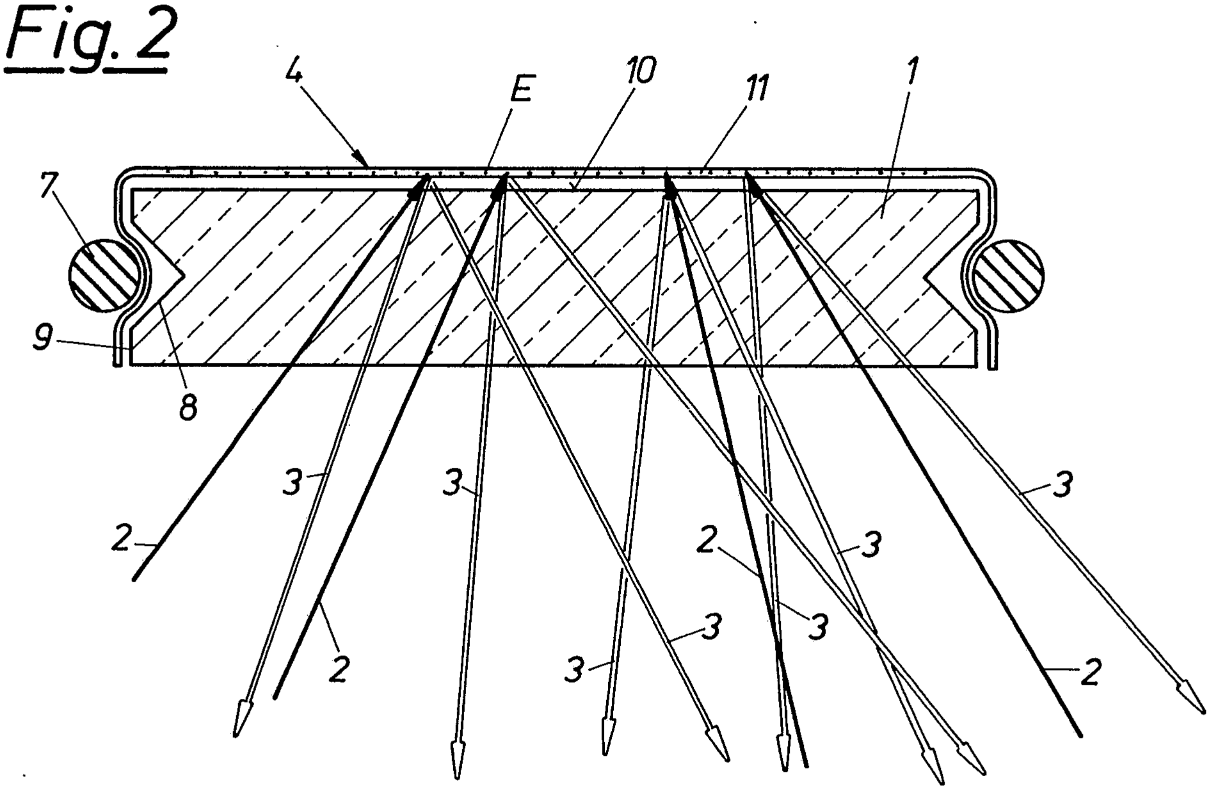

Fig. 3a

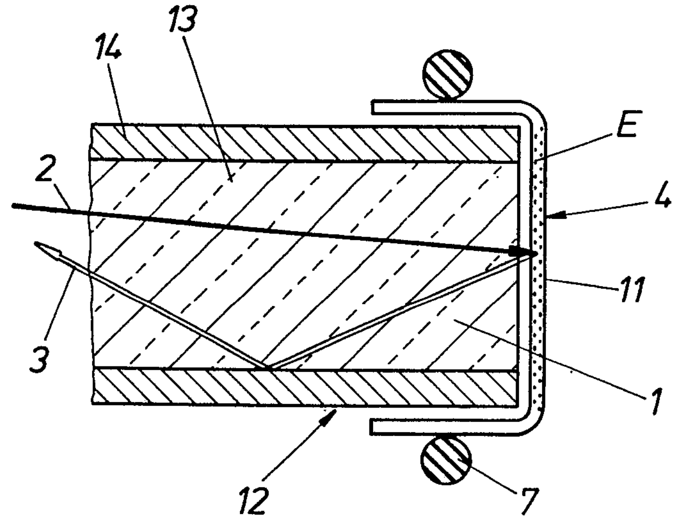


Fig. 3b

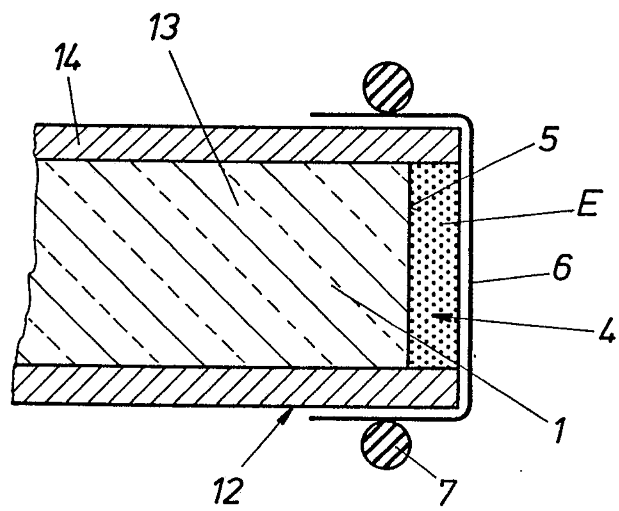


Fig. 3c

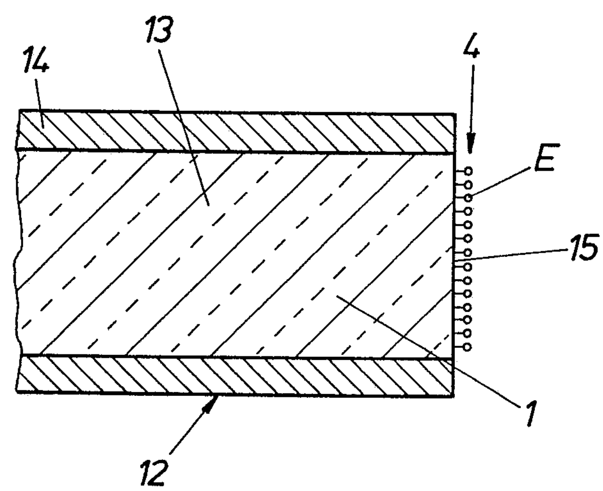

Fig. 4

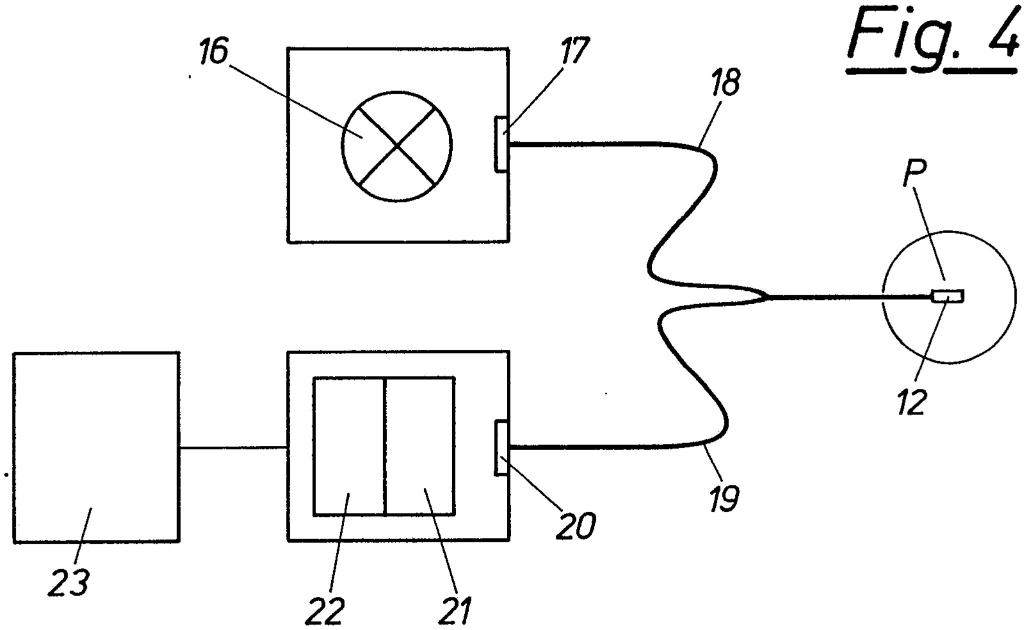


Fig. 5

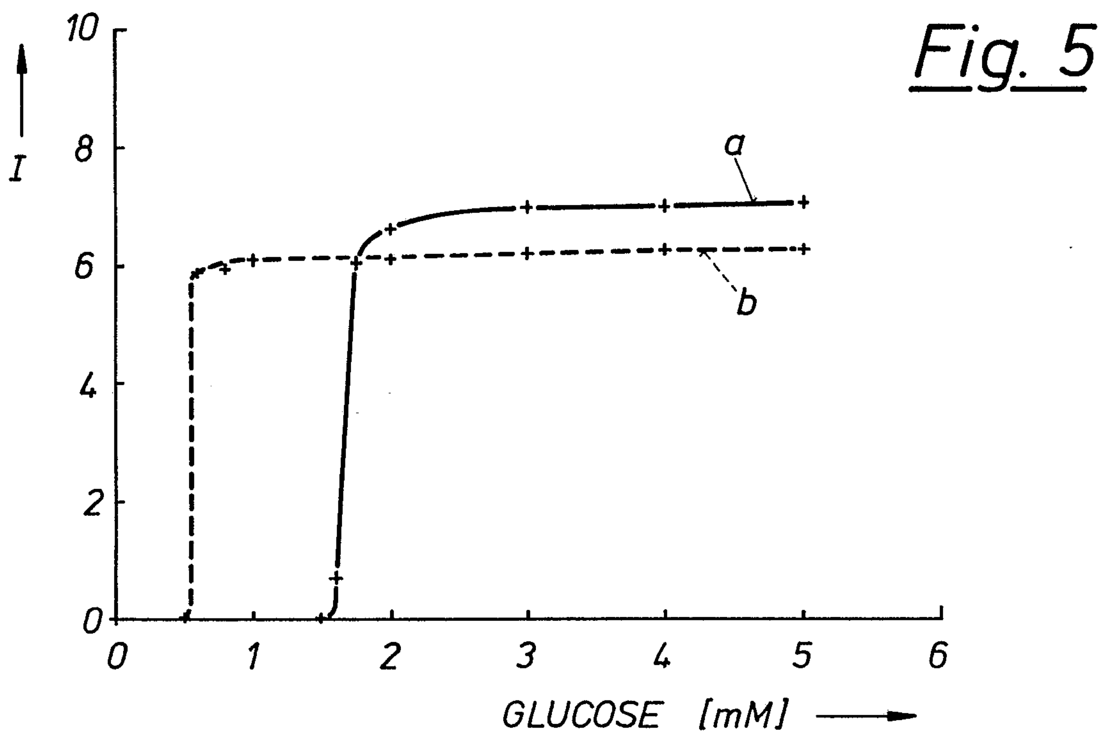

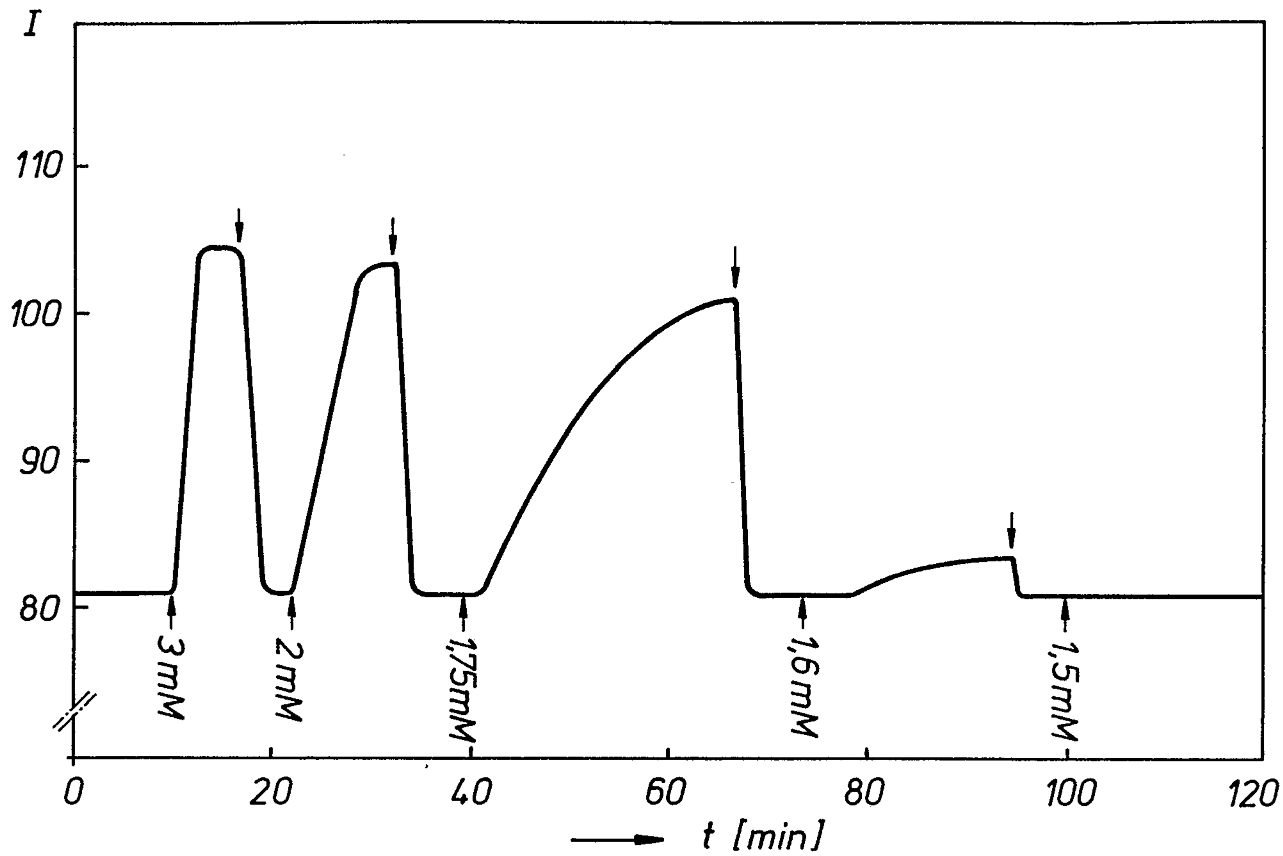
I
110
100
90
80
3mM
2mM
1,75mM
1,6mM
1,5mM
0
20
40
60
80
100
120
t [min]

Fig. 6

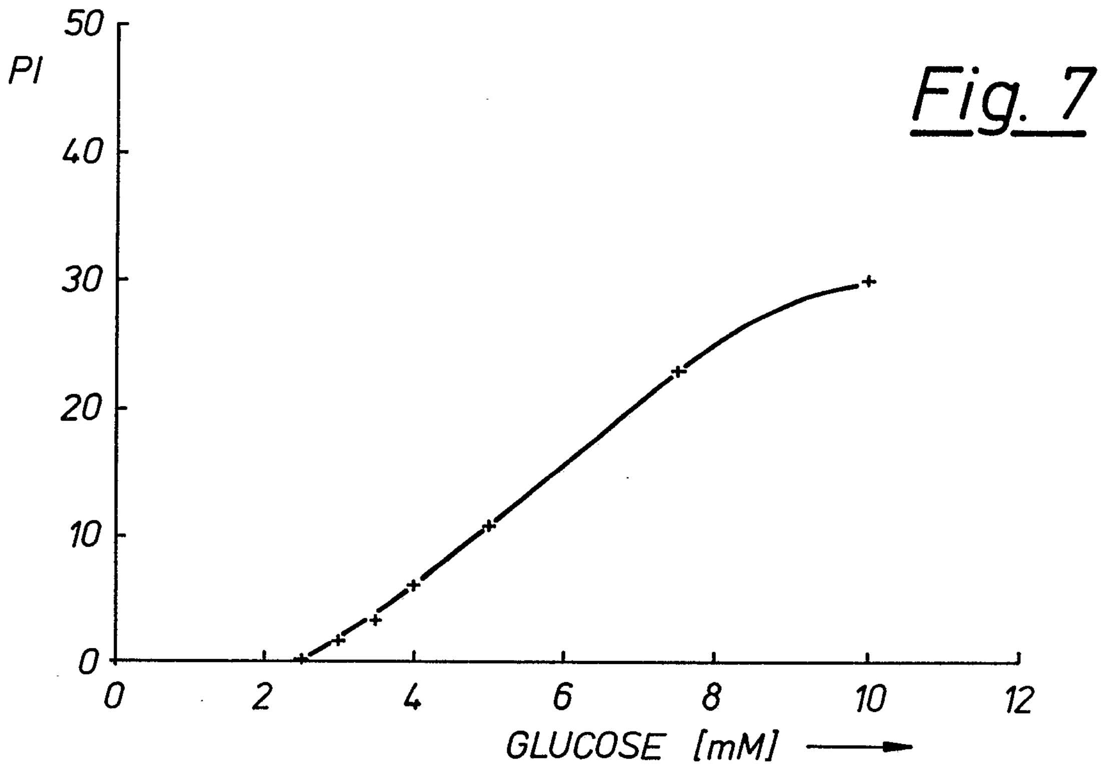
PI
50
40
30
20
10
0
0
2
4
6
8
10
12
GLUCOSE [mM]

Fig. 7